# EUROPÄISCHE PATENTSCHRIFT

(11) **EP 0 755 914 B1**
(45) Veröffentlichungstag und Bekanntmachung des Hinweises auf die Patenterteilung: **06.10.1999**
(21) Anmeldenummer: 96111158.0
(22) Anmeldetag: 11.07.1996
(51) Int. Cl.: C07C 63/15, C07C 63/16, C07C 51/377

(54) **Verfahren zur Herstellung von Isobenzofurandionen**
Process for producing isobenzofurandiones
Procédé de préparation d'isobenzofuranediones

(30) Priorität: 24.07.1995 DE 19526923
(43) Veröffentlichungstag der Anmeldung: 29.01.1997
(73) Patentinhaber: BAYER AG, 51368 Leverkusen (DE)
(72) Erfinder: Käsbauer, Josef, Dr., 42929 Wermelskirchen (DE); Fiege, Helmut, Dr., 51373 Leverkusen (DE)

(56) Entgegenhaltungen:
- PATENT ABSTRACTS OF JAPAN vol. 11, no. 379 (C-463) [2826] , 10.Dezember 1987 & JP-A-62 148450 (KAWASAKI KASEI CHEM.LTD), 2.Juli 1987,

## Beschreibung

Die vorliegende Erfindung betrifft ein verbessertes Verfahren zur Herstellung von Isobenzofurandionen durch Dehydrierung von Tetrahydroisobenzofurandionen in Gegenwart von Palladium- und/oder Platin-Trägerkatalysatoren.

Die Aromatisierung von Tetrahydroisobenzofurandionen zu Isobenzofurandionen über Abspaltungsreaktionen wie Dehydrierung oder Hydrodehalogenierung ist bekannt. Von besonderem Interesse ist die Dehydrierung von 5-Methyl-tetrahydroisobenzofüran-1,3-dion zu 5-Methyl-isobenzofuran-1,3-dion, einem Vorprodukt für Pflanzenschutzmittel (siehe z.B. EP-A-1 158 000).

Bei der Katalyse mit Palladium auf Aluminiumoxid als Trägermaterial tritt bei 300°C in Abwesenheit eines Wasserstoffakzeptors neben der Dehydrierung auch die Hydrierung zum Hexahydro-Produkt auf (siehe C.A. 105:97266, 106:102704 und 109:92690).

Eine Verbesserung der Selektivität wurde bei anderen Edukten durch Zusatz von Wasserstoffakzeptoren erreicht. So kann man bei der Palladium-katalysierten Dehydrierung von 4-Methyl-tetrahydrophthalsäurediethylester bei 210°C und beim Zusatz von Maleinsäurediethylester (siehe C.A. 108:37393e) eine Ausbeute an 4-Methylphthalsäurediethylester von 86,7 % der Theorie erreichen. Es ist anzunehmen, daß sich das Hexahydroderivat in einer Ausbeute von 13 % der Theorie bildet, da dieses im Vergleichsversuch ohne Mäleinesterzusatz mit einer Ausbeute von 56 % der Theorie entsteht. Das Verfahren hat den Nachteil, daß Maleinsäurediethylester in einem Überschuß eingesetzt werden muß (240 mol%) und sich aus dem Überschuß bei den Reaktionstemperaturen Fumarate bilden. Fumarate stören durch ihr Sublimationsverhalten bei der Aufarbeitung und müssen vom durch Wasserstoffaufnahme entstehenden Bernsteinsäurediethylester abgetrennt werden.

Ein Zusatz von Propylen bei der Dehydrierung von Methyl-tetrahydroisobenzofurandion-1,3 führte zu einer Ausbeute von 85 % der Theorie an gewünschtem Produkt (siehe C.A. 110:213398). Das Einleiten von Propylen in eine Schmelze, die eine Temperatur von ca. 200°C aufweist, ist jedoch im technischen Maßstab sicherheitstechnisch sehr aufwendig und daher äußerst unökonomisch. Außerdem ist die Ausbeute auch noch hier verbesserungsbedürftig.

Aufgabe der vorliegenden Erfindung war es deshalb, ein Verfahren bereitzustellen, das die Nachteile des Standes der Technik überwindet und es gestattet, auf technisch einfache Weise Isobenzofurandione durch katalytische Dehydrierung in hohen Ausbeuten herzustellen.

Es wurde nun ein Verfahren zur Herstellung von Isobenzofurandionen durch Dehydrierung von Tetrahydroisobenzofurandionen in Gegenwart von Katalysatoren bei erhöhter Temperatur gefunden, das dadurch gekennzeichnet ist, daß man destillierte Tetrahydroisobenzofurandione in Gegenwart von Palladium- und/oder Platin-Trägerkatalysatoren und in Gegenwart von Malein- oder Fumarsäurederivaten in einem Rieselphasen- oder Sumpfphasenverfahren auf Temperaturen zwischen 120 und 300°C erhitzt, wobei man beim Sumpfphasenverfahren oberhalb 80 bis 110°C die Temperatur mit einer Rate von 0,6 bis 3°C pro Minute ansteigen läßt.

In das erfindungsgemäße Verfahren kann man dann z.B. Tetrahydroisobenzofurandione der Formel (I) einsetzen 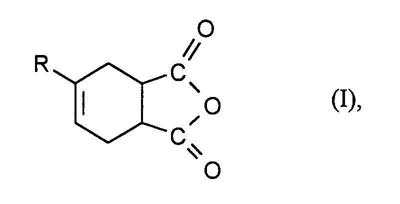 in der
- R: für Wasserstoff oder C₁-C₆-Alkyl steht. Vorzugsweise steht R für Methyl.

Verbindungen der Formel (I) sind durch eine Diels-Alder-Synthese aus Maleinsäureanhydrid und den entsprechenden Diolefinen gut zugänglich. Wenn man nichtdestillierte Tetrahydroisobenzofurandione einsetzt, verlangsamt sich die Reaktionsgeschwindigkeit und erniedrigt sich die erzielbare Ausbeute (siehe Vergleichsbeispiel 2).

Als Katalysatoren kommen z.B. an sich bekannte Palladium- und/oder Platin-Trägerkatalysatoren in Frage, beispielsweise solche, die 0,1 bis 10 Gew.-% Palladium und/oder Platin auf Aluminiumoxid, Siliziumdioxid, Kieselsäure, Kieselgur, Silikaten oder Kohle enthalten. Bevorzugt enthalten die Katalysatoren 1 bis 8 Gew.-% Palladium und/oder Platin auf Aluminiumoxid oder Aktivkohle. Besonders bevorzugt sind Palladium-Trägerkatalysatoren.

Das Palladium und/oder Platin kann nicht nur in metallischer Form sondern auch in Form von Verbindungen eingesetzt werden. Im allgemeinen werden solche Verbindungen unter den Reaktionsbedingungen zum Metall reduziert.

Der Katalysator gelangt vorzugsweise in möglichst wasserfreier Form zum Einsatz. Palladium-Trägerkatalysatoren sind häufig nur in wasserhaltiger Form im Handel erhältlich. Solche wasserhaltigen Katalysatoren kann man vom Wasser befreien, indem man sie zunächst mit einem mit Wasser mischbaren Lösungsmittel, z.B. einem niedrigen Alkohol, wäscht und anschließend mit einem systemimmanenten Produkt behandelt, z.B. einem Bernsteinsäurederivat wie Dimethylsuccinat. Wenn Wasser im Reaktionssystem zugegen ist, führt dieses zur Spaltung der Anhydridgruppe im eingesetzten Tetrahydroisobenzofurandion und/oder im gebildeten Isobenzofurandion und damit zu unerwünschter Nebenproduktbildung.

Den Katalysator kann man bei Durchführung des erfindungsgemäßen Verfahrens in einer Sumpffahrweise beispielsweise in Mengen von 1 bis 10 Gew.-% Palladium und/oder Platin, bezogen auf das Edukt, einsetzen. Vorzugsweise beträgt diese Menge 2 bis 8 Gew.-%. Bei einer Rieselphasenfahrweise kann man z.B. über einen Liter Trägerkatalysator 50 bis 300 ml Einsatzgemisch pro Stunde leiten.

Als Malein- und Fumarsäurederivate kommen z.B. Maleinsäureanhydrid, Maleinimide, Maleinamidsäure, Maleinsäurealkylester und Fumarsäurealkylester in Frage. Bevorzugt sind Maleinsäureanhydrid, Maleinsäuredimethyl- und -diethylester und Fumarsäuredimethyl- und -diethylester, ganz besonders bevorzugt sind Maleinsäuredimethylester und Fumarsäuredimethylester.

Das jeweilige Malein- oder Fumarsäurederivat kann beispielsweise in einer Menge von 180 bis 220 mol%, bezogen auf das Edukt, eingesetzt werden. Vorzugsweise liegt diese Menge bei 190 bis 210 mol%, insbesondere bei 195 bis 205 mol%.

Das erfindungsgemäße Verfahren wird bei Temperaturen von 120 bis 300°C durchgeführt. Vorzugsweise arbeitet man bei 130 bis 250°C. Bei der Sumpffahrweise ist in der ersten Erhitzungsphase von Raumtemperatur bis zu 80 bis 110°C die Steigungsrate der Temperatur ohne besondere Bedeutung. Ab 80 bis 110°C läßt man die Temperatur mit einer Rate von 0,6 bis 3°C pro Minute, vorzugsweise 0,8 bis 2,0°C pro Minute ansteigen. Bei Temperatursteigerungsraten unter 0,6°C pro Minute fällt die erzielbare Ausbeute ab (siehe Vergleichsbeispiel 1). Temperatursteigerungsraten von mehr als 3°C pro Minute würden im technischen Maßstab einen unökonomisch hohen apparativen Aufwand erfordern.

Der Druck wahrend der Reaktion ist nicht kritisch und kann z.B. bei 1 bis 10 bar, vorzugsweise bei Normaldruck bis 3 bar, liegen. Besonderes bevorzugt arbeitet man bei Temperaturen bis 210°C bei Normaldruck.

Bei ansatzweiser Durchführung des erfindungsgemäßen Verfahrens ist die Reaktion im allgemeinen nach 1 bis 4 Stunden beendet. Für die Rieselphasenfahrweise sind die oben angegebenen Katalysatorbelastungen so, daß ein praktisch vollständiger Umsatz bei einem Durchgang erzielt wird.

Das nach Beendigung der Reaktion vorliegende Gemisch kann man beispielsweise wie folgt aufarbeiten: Zunächst trennt man gegebenenfalls vorhandenen Katalysator ab und entfernt dann das aus dem Malein- oder Fumarsäurederivat entstandene Bernsteinsäurederivat, z.B. durch Destillation bei vermindertem Druck. Das gebildete Isobenzofurandion, welches beispielsweise der Formel (II) entspricht 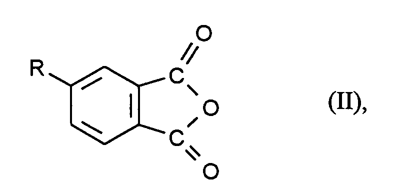 in der R die bei Formel (I) angegebene Bedeutung hat, hinterbleibt dann häufig in so reiner Form, daß man es direkt weiterverwenden kann. Erforderlichenfalls kann man es weiter reinigen, z.B. durch Destillation.

Das erfindungsgemäße Verfahren hat eine Reihe von überraschenden Vorteilen. So war anzunehmen, daß bei den hohen Reaktionstemperaturen Malein- und Fumarsäurederivate mit der Anhydridgruppe des Tetrahydroisobenzofurandions reagieren. Diese Reaktionsmöglichkeit ist beim Verfahren gemäß C.A. 108:37393e ausgeschlossen, da dort nur Ester eingesetzt werden und nicht, wie erfindungsgemäß, wenigstens ein Anhydrid.

Weiterhin war zu erwarten, daß das Malein- und Fumarsäurederivat als Dienophil mit dem Zwischenprodukt aus der Dehydrierung, dem Dihydroderivat mit Dienstruktur, in einer Diels-Alder-Reaktion reagiert.

Übertaschenderweise treten beide Nebenreaktionen nur in zu vernachlässigendem Maße auf und das gewünschte Dehydrierprodukt wird im erfindungsgemäßen Verfahren mit Ausbeuten erhalten, die weit über den bisher erzielbaren von 85 bis 87 % der Theorie liegen, nämlich im allgemeinen bei über 95 % der Theorie.

Beim erfindungsgemäßen Verfahren müssen keine Gase gehandhabt werden, so daß kein Aufwand für deren Sicherheitstechnik und Entsorgung anfallt.

Die als Koppelprodukte gebildeten Bernsteinsäurederivate stellen wertvolle chemische Zwischenprodukte dar, die als biologisch gut abbaubare Verbindungen für vielfältige Zwecke Verwendung finden können, z.B. als Lösungsmittel, als Komponenten zur Herstellung von Polyestern und als Zwischenprodukte für organische Pigmente.

Schließlich ist überraschend, daß nicht nur die gewünschten Isobenzofurandione in hoher Ausbeute und Selektivität entstehen, sondern ebenfalls die Folgeprodukte der Wasserstoffaufnahme, nämlich die Bernsteinsäurederivate. Im erfindungsgemäßen Verfahren entstehen praktisch keine Abfallstoffe, die zu entsorgen wären. Das erfindungsgemäße Verfahren ist deswegen ökologisch besonders vorteilhaft.

### Beispiele

### Beispiel 1

In einem Reaktionsgefäß wurden 144 g Maleinsäuredimethylester und 83 g destilliertes 5-Methyltetrahydroisobenzofurandion-1,3 bei Raumtemperatur vorgelegt.

7,7 g eines käuflichen Katalysators der 5 Gew.-% Palladium auf Kohle enthielt und einen Wassergehalt von 52 Gew.-% aufwies, wurden in Methanol suspendiert, abgesaugt, mit Methanol gewaschen, anschließend mit Dimethylsuccinat versetzt und nochmals mit Dimethylsuccinat gewaschen. Dieser mit Dimethylsuccinat befeuchtete, wasserfreie Katalysator wurde dem oben angegebenen Ansatz zugegeben. Unter leichtem Stickstoffstrom wurde dann unter Rühren auf 110 bis 120°C aufgeheizt und mit einer Temperatursteigerung von 1°C pro Minute weiter auf 205°C erhitzt. Bei 200 bis 205°C wurde 1,5 Stunden nachgerührt. Anschließend wurde das Reaktionsgemisch abgekühlt, der Katalysator abgesaugt und das Filtrat einer Destillation unterworfen. Bei 30 mbar wurde Dimethylsuccinat mit einem Siedepunkt von 96 bis 98°C abdestilliert (Reinheit über 99,3 Gew.-%). Der Destillationsrückstand wies einen Gehalt von 5-Methyl-isobenzofurandion-1,3 von über 96 Gew.-% auf. Die Ausbeute betrug 97 % der Theorie. Das 5-Methylisobenzofurandion-1,3 wurde destilliert und mit einem Siedepunkt von 177°C bei 30 mbar in sehr reiner Form erhalten.

### Beispiel 2

In einem Rieselphasenreaktor, der mit 100 ml tablettenförmigem Katalysator (5 Gew.-% Palladium auf Aluminiumoxid) gefüllt war, wurden bei 200°C stündlich 10 ml einer Lösung aus 1 mol 5-Methyl-tetrahydroisobenzofurandion-1,3 und 2 mol Maleinsäuredimethylester von oben zugepumpt. Nach Passieren des Reaktors war der Maleinsäuredimethylester zu 100 % umgesetzt. In dem den Reaktor verlassenden Produktgemisch konnte kein Fumarsäuredimethylester nachgewiesen werden. 5-Methylisobenzofurandion-1,3 hatte sich mit einer Selektivität von über 95 % gebildet, Dimethylsuccinat mit einer Selektivität von über 97 %. Der Dehydrier-Umsatz lag bei 99,8 %. Der Katalysator zeigte nach 500 Betriebsstunden keine Zeichen von Desaktivierung.

### Vergleichsbeispiel 1

Es wurde verfahren wie bei Beispiel 1, jedoch wurde ab 120°C mit einer Aufheizrate von 0,5°C pro Minute weiter erhitzt. Die Ausbeute an 5-Methyl-isobenzofurandion-1,3 erniedrigte sich dadurch auf 94 % der Theorie.

### Vergleichsbeispiel 2

Es wurde verfahren wie bei Beispiel 1, jedoch wurde 5-Methyltetrahydroisobenzofurandion-1,3 eingesetzt, das nicht destilliert worden war. Die Reaktionsgeschwindigkeit erniedrigte sich dadurch deutlich. Nach 4stündigem Rühren bei 205°C lag der Umsatz von 5-Methyltetrahydroisobenzofurandion-1,3 erst bei 95 % und die Ausbeute an 5-Methyl-isobenzofurandion-1,3 erst bei 92 % der Theorie.

## Patentansprüche

1. Verfahren zur Herstellung von Isobenzofurandionen durch Dehydrierung von Tetrahydroisobenzofurandionen in Gegenwart von Katalysatoren bei erhöhter Temperatur, dadurch gekennzeichnet, daß man destillierte Tetrahydroisobenzofurandione in Gegenwart von Palladium- und/oder Platin-Trägerkatalysatoren und in Gegenwart von Malein- und Fumarsäurederivaten in einem Rieselphasen- oder Sumpfphasenverfahren auf Temperaturen zwischen 120 und 300°C erhitzt, wobei man beim Sumpfphasenverfahren oberhalb 80 bis 110°C die Temperatur mit einer Rate von 0,6 bis 3°C pro Minute ansteigen läßt.

2. Verfahren nach Anspruch 1, dadurch gekennzeichnet, daß man Tetrahydroisobenzofurandione der Formel (I) einsetzt 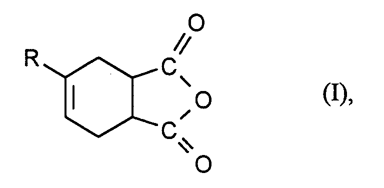 in der
R für Wasserstoff oder C₁-C₆-Alkyl steht,
als Malein- oder Fumarsäurederivate Maleinsäureanhydrid, Maleinimide, Maleinamidsäure, Maleinalkylester oder Fumarsäurealkylester einsetzt und Isobenzofurandione der Formel (II) erhält 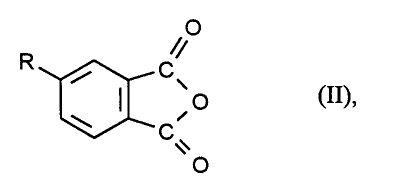 in der R die bei Formel (I) angegebene Bedeutung hat.

3. Verfahren nach Ansprüchen 1 und 2, dadurch gekennzeichnet, daß man als Katalysatoren solche einsetzt, die 0,1 bis 10 Gew.-% Palladium und/oder Platin auf Aluminiumoxid, Siliziumdioxid, Kieselsäure, Kieselgur, Silikaten oder Kohle enthalten.

4. Verfahren nach Ansprüchen 1 bis 3, dadurch gekennzeichnet, daß man es als Sumpfphasenverfahren durchführt und 1 bis 10 Gew.-% Palladium und/oder Platin, bezogen auf das Edukt, einsetzt.

5. Verfahren nach Ansprüchen 1 bis 3, dadurch gekennzeichnet, daß man es als Rieselphasenverfahren durchführt und über 1 l Trägerkatalysator 50 bis 300 ml Einsatzgemisch pro Stunde leitet.

6. Verfahren nach Ansprüchen 1 bis 5, dadurch gekennzeichnet, daß man das Malein- oder Fumarsäurederivat in einer Menge von 180 bis 220 mol%, bezogen auf das Edukt, einsetzt.

7. Verfahren nach Ansprüchen 1 bis 6, dadurch gekennzeichnet, daß man beim Sumpfphasenverfahren oberhalb 80 bis 110°C die Temperatur mit einer Rate von 0,8 bis 2,0°C pro Minute ansteigen läßt.

8. Verfahren nach Ansprüchen 1 bis 7, dadurch gekennzeichnet, daß man es bei Drucken im Bereich 1 bis 10 bar durchführt.

9. Verfahren nach Ansprüchen 1 bis 8, dadurch gekennzeichnet, daß man das nach der Reaktion vorliegende Gemisch aufarbeitet, indem man zunächst gegebenenfalls vorhandenen Katalysator abtrennt und dann das aus dem Malein- oder Fumarsäurederivat entstandene Bernsteinsäurederivat durch Destillation bei vermindertem Druck abdestilliert.

## Claims

1. Process for the preparation of isobenzofurandiones by the dehydrogenation of tetrahydroisobenzofurandiones in the presence of catalysts at elevated temperature, characterized in that distilled tetrahydroisobenzofurandiones are heated to temperatures of between 120 and 300°C in the presence of supported palladium and/or platinum catalysts and in the presence of maleic and fumaric acid derivatives in a trickle-phase or liquid-phase procedure, the temperature in the liquid-phase procedure being increased at a rate of 0.6 to 3°C per minute above 80 to 110°C.

2. Process according to Claim 1, characterized in that tetrahydroisobenzofurandiones of the formula (I) 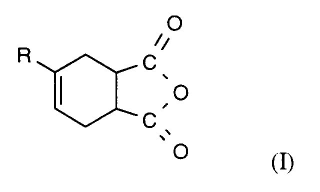 are used, in which
R represents hydrogen or C₁-C₆-alkyl,
maleic anhydride, maleimides, maleamic acid, alkyl maleates or alkyl fumarates are used as maleic or fumaric acid derivatives, and isobenzofurandiones of the formula (II) 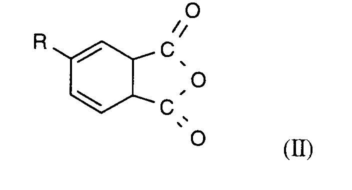 are obtained, in which R is as defined for the formula (I).

3. Process according to Claims 1 and 2, characterized in that the catalysts used contain 0.1 to 10% by weight of palladium and/or platinum on aluminium oxide, silicon dioxide, silicic acid, kieselguhr, silicates or charcoal.

4. Process according to Claims 1 to 3, characterized in that it is carried out as a liquid-phase process using 1 to 10% by weight of palladium and/or platinum, based on the starting material.

5. Process according to Claims 1 to 3, characterized in that it is carried out as a trickle-phase process, 50 to 300 ml/hour of starting mixture being passed over 1 l of supported catalyst.

6. Process according to Claims 1 to 5, characterized in that the maleic or fumaric acid derivative is used in an amount of 180 to 220 mol%, based on the starting material.

7. Process according to Claims 1 to 6, characterized in that the temperature in the liquid-phase procedure is increased at a rate of 0.8 to 2.0°C per minute above 80 to 110°C.

8. Process according to Claims 1 to 7, characterized in that it is carried out at pressures in the range 1 to 10 bar.

9. Process according to Claims 1 to 8, characterized in that the mixture present after the reaction is worked up by a process in which any catalyst present is first separated off and the succinic acid derivative formed from the maleic or fumaric acid derivative is then distilled off by distillation at reduced pressure.

## Revendications

1. Procédé pour la préparation d'isobenzofurannediones par déshydrogénation de tétrahydroisobenzofurannediones en présence de catalyseurs à une température élevée, caractérisé en ce qu'on chauffe à des températures entre 120 et 300°C des tétrahydroisobenzofurannediones distillées, en présence de catalyseurs de palladium et/ou de platine déposés sur un support et en présence de dérivés de l'acide maléique et de l'acide fumarique, dans un procédé en phase de ruissellement ou en phase liquide, en faisant en sorte, dans le procédé en phase liquide, qu'au-delà de 80°C à 110°C, on élève la température à raison de 0,6 à 3°C par minute.

2. Procédé selon la revendication 1, caractérisé en ce qu'on met en oeuvre des tétrahydroisobenzofurannediones répondant à la formule (I) 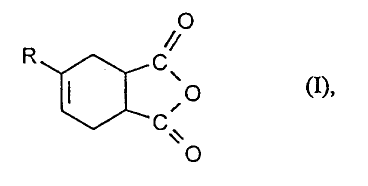 dans laquelle
R représente un atome d'hydrogène ou un groupe alkyle en C₁-C₆,
à titre de dérivés de l'acide maléique ou de l'acide fumarique, on met en oeuvre l'anhydride maléique, des maléimides, l'acide maléamique, des esters alkyliques de l'acide maléique ou des esters alkyliques de l'acide fumarique et on obtient des isobenzofurannediones répondant à la formule (II) 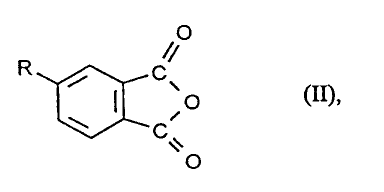 dans laquelle R a la signification indiquée à la formule (I).

3. Procédé selon les revendications 1 et 2, caractérisé en ce qu'on met en oeuvre, à titre de catalyseurs, ceux qui contiennent du palladium et/ou du platine à concurrence de 0,1 à 10% en poids sur de l'oxyde d'aluminium, du dioxyde de silicium, de l'acide silicique, du kieselgur, des silicates ou du charbon.

4. Procédé selon les revendications 1 à 3, caractérisé en ce qu'on l'effectue sous la forme d'un procédé en phase liquide et on met en oeuvre du palladium et/ou du platine à concurrence de 1 à 10% en poids rapportés à l'éduit.

5. Procédé selon les revendications 1 à 3, caractérisé en ce qu'on l'effectue sous la forme d'un procédé en phase de ruissellement et on guide par-dessus 1 litre de catalyseur déposé sur un support de 50 à 300 ml du mélange de mise en oeuvre par heure.

6. Procédé selon les revendications 1 à 5, caractérisé en ce qu'on met en oeuvre le dérivé de l'acide maléique ou de l'acide fumarique en une quantité de 180 à 220 moles % rapportés à l'éduit.

7. Procédé selon les revendications 1 à 6, caractérisé en ce que, dans le procédé en phase liquide, au-delà de 80°C jusqu'à 110°C, on fait en sorte d'élever la température à raison de 0,8 à 2,0°C par minute.

8. Procédé selon les revendications 1 à 7, caractérisé en ce qu'on l'effectue sous des pressions dans le domaine de 1 à 10 bar.

9. Procédé selon les revendications 1 à 8, caractérisé en ce qu'on traite le mélange présent après la réaction en séparant d'abord le catalyseur éventuellement présent, puis en éliminant par distillation sous pression réduite le dérivé de l'acide succinique obtenu à partir du dérivé de l'acide maléique ou de l'acide fumarique.
